Europäisches Patentamt

(19) European Patent Office

Office européen des brevets

(11) EP 0 565 361 B1

(12) **EUROPEAN PATENT SPECIFICATION**

(45) Date of publication and mention
of the grant of the patent:
**10.07.1996 Bulletin 1996/28**

(51) Int. Cl.$^6$: **A61K 9/127**

(21) Application number: **93302734.4**

(22) Date of filing: **07.04.1993**

(54) **Liposome formulation and process for production thereof**

Liposomenformulierung und deren Herstellungsverfahren

Formulation liposomique et son procédé de fabrication

(84) Designated Contracting States:
**CH DE FR GB IT LI NL**

(30) Priority: **07.04.1992 JP 114086/92**

(43) Date of publication of application:
**13.10.1993 Bulletin 1993/41**

(73) Proprietor: **BANYU PHARMACEUTICAL CO., LTD.**
**Chuo-ku Tokyo 103 (JP)**

(72) Inventors:
• **Sawahara, Hideyuki,**
**c/o Banyu Pharmaceutical Co.**
**Menuma-machi, Osato-gun, Saitama (JP)**
• **Shibata, Masahiko,**
**c/o Banyu Pharmaceutical Co.**
**Menuma-machi, Osato-gun, Saitama (JP)**
• **Tsunemi, Shinobu,**
**c/o Banyu Pharmaceutical Co.**
**Menuma-machi, Osato-gun, Saitama (JP)**
• **Funada, Satoru,**
**c/o Banyu Pharmaceutical Co.**
**Menuma-machi, Osato-gun, Saitama (JP)**
• **Inoue, Kimio,**
**c/o Banyu Pharmaceutical Co.**
**Menuma-machi, Osato-gun, Saitama (JP)**
• **Ishimaru, Sueaki,**
**c/o Banyu Pharmaceutical Co.**
**Menuma-machi, Osato-gun, Saitama (JP)**

(74) Representative: **Davies, Jonathan Mark**
**Reddie & Grose**
**16 Theobalds Road**
**London WC1X 8PL (GB)**

(56) References cited:
**EP-A- 0 331 504**

• **G. GREGORIADIS 'liposome technology volume
III' 1984 , CRC PRESS, INC. , BOCA RATON (US)**
• **JOURNAL OF PHARMACEUTICAL SCIENCES
vol. 80, no. 6, June 1991, WASHINGTON (US)
pages 522 - 525 , XP206524 K. IGA ET AL.
'increased tumor cisplatin levels in heated
tumors in mice after administration of
thermosensitive, large unilamellar vesicles
encapsulating cisplatin'**

**Description**

BACKGROUND OF THE INVENTION

1. Field of the Invention

The present invention relates to a liposome formulation in which a pharmacologically active ingredient is entrapped in the liposomes, and a process for production thereof.

2. Related Art

As drug delivery systems, liposome formulations with various properties have been developed for targeting therapy of cancer sites. One of said known systems is a temperature-sensitive liposome formulation, which can be used in combination with hyperthermia therapy of cancer to enhance the effects of chemotherapy. Note, hyperthermia therapy is therapy wherein cancer cells are specifically injured by locally heating a cancer site (to about 43°C) using an appropriate apparatus. See, for example, J. N. Weinstein, R. L. Magin, M. B. Yatvin and D. S. Zaharko, Science, 204. 188 (1979); J. N. Weinstein, R. R. Magin, R. L. Cysyk and D. S. Zaharko, Cancer Res. 40, 1388 (1980); M. B. Yatvin, T. C. Cree, I. M. Tegmo-Larsson and J. J. Gipp., Strahlentherapie 160, 732 (1984).

A liposome formulation is intravenously administerted and reaches a cancer site carried by the blood. When the cancer site is heated, temperature-sensitive liposomes detect the difference between the temperature of the heated site and the normal body temperature and are disrupted to locally release a drug. Since temperature-sensitive liposomes are stable in normal tissues not heated and therefore do not release the drug there, their reduced side effects are expected. Such a temperature-sensitive liposome formulation enhances the targeting effect of chemotherapeutic drugs.

As described above, it is considered that application of a temperature-sensitive liposome formulation is useful to hyperthermia therapy of cancer. Conventional liposome formulations use large unilamellar vesicles (LUV); generally having a diameter of 0.1 to 2.0 µm), but are considered to have two drawbacks. One of the drawbacks is that since the LUVs intravenously administered are easily absorbed into reticuloendotherial tissues, the chemotherapeutic drug (generally considered to have side effects) may adversely affect normal tissues. Another drawback is that since the LUVs are easily absorbed into reticuloendotherial tissues, it is difficult to maintain their desired concentration in the blood, so that the targeting efficiency to a cancer site is low. In a temperature-sensitive liposome system, it is important to maintain the blood level as high as possible. As A. Gabizon et al. (Proc. Natl. Acad. Sci. 85, 6949, 1988) pointed out, it is known that liposomes remaining for a long time in the circulating blood have a tendency of passively accumulating at a cancer site, and therefore a higher synergistic effect for targeting is expected.

As a hint to solve the above-mentioned problems, it is known that as the particle size of liposomes becomes smaller, incorporation into the reticuloendotherial tissues becomes to be diminished and their concentration in the blood can be maintained at a high level. See, for example, R. L. Juliano and D. Stamp, Biochem. Biophys. Res. Commun., 63, 651 (1975); T. M. Allen and J. M. Everest, J. Pharmacol. Exp. Ther., 226, 539 (1983); Y. Sato, H. Kiwada and Y. Kato, Chem. Pharm. Bull., 34, 4244 (1986); R. M. Abra and C. A. Hunt, Biochim. Biophys. Acta, 666, 493 (1981); H. Kiwada, S. Obara, H. Nishiwaki and Y. Kato, Chem. Pharm. Bull., 32, 1249 (1986); C. A. Hunt, Y. M. Rustum, E. Mayhew and D. Papahadjopoulos, Drug. Metab. Dispos., 7, 124 (1979), and Y. E. Rahman, E. A. Cerny, K. R. Patel, E. H. Lau and B. J. Wright, Life Sci., 31, 2061 (1982). However, if the particle size becomes smaller, the temperature sensitivity drastically drops. See, for example, Int. J. Pharmaceut., 57, 241 (1989); Cancer Drug. Delivery 3, 223 (1986); and Y. Ogawa and H. Toguchi, Gan To Kagakuryo Ho (Cancer and Chemotherapy) 17, 1127 (1990).

Accordingly, at present, it is desired to develop a liposome formulation having a liposome particle size small enough to maintain a desired concentration in the blood and having excellent temperature sensitivity.

SUMMARY OF THE INVENTION

Accordingly, the present invention provides a liposome formulation comprising liposome entrapping a drug-containing solution, having an osmotic pressure 2.0 to 5.0 times higher than the physiological osmotic pressure, said liposomes being in small unilamellar vesicle having an average particle diameter of not more than 0.1 µm and having a phase transition temperature of the membrane of 40°C to 45°C.

The present invention further provides a process of production of a liposome formulation, comprising the steps of:

(1) preparing a drug-containing solution having an osmotic pressure 2.0 to 5.0 times higher than the physiological osmotic pressure;
(2) preparing a thin layer of a phospholipid;
(3) mixing the drug-containing solution and the thin layer of phospholipid to form multilamellar vesicle liposomes; and

(4) sonicating the multilamellar vesicle liposomes to form small Unilamellar Liposomes comprising liposomes entrapping the drug-containing solution having an osmotic pressure 2.0 to 5.0 times higher than the physiological osmotic pressure, said small unilamellar vesicles having an average particle diameter of not more than 0.1 μm and having a phase transition temperature of the membrane of 40°C to 45°C.

BRIEF EXPLANATION OF DRAWINGS

Figure 1 shows the change of the releasing ratio of calcein entrapped in liposomes prepared in Example 3 at different temperatures along with the elapse of time, and

Fig. 2 shows an anticancer effect of a liposome formulation entrapping therein CDDP prepared in the examples in comparison with comparative examples.

DETAILED DESCRIPTION OF THE INVENTION

First, the composition of the membrane forming the present liposome formulation will be explained. According to the present invention, the components of the liposome membrane and the-ratios thereof are chosen so that the phase transition temperature of the membrane formed from the components is 40°C to 45°C, preferably 40°C to 43°C. As components of the membrane, various kinds of phospholipids whose acyl groups are saturated are preferably used singly or in combination. Two saturated acyl groups of glycerol phospholipid are those derived from saturated higher fatty acids. Usually $C_{12}$-$C_{20}$ saturated acyl groups and preferably those $C_{14}$-$C_{18}$ saturated acyl groups are used. Such phospholipids are, for example, hydrogenized lecithin such as hydrogenized yolk or soybean lecithin, and synthetic or semi-synthetic phosphatidyl choline comprising a combination of lauroyl, myristoyl, palmitoyl or stearoyl groups, or the like. In addition, depending on the nature of drugs entrapped, phosphatidylglycerol having saturated acyl groups can be used. Particularly, synthetic or semi-synthetic phosphatidyl cholines are preferably used. Among them, dipalmitoyl phosphatidyl choline having C16 saturated acyl groups (DPPC: phase transition temperature 41.4°C) or distearoyl phosphatidylcholine having C18 saturated acyl groups (DSPC: phase transition temperature 54.9°C) is preferably used because the phase transition temperatures thereof are near to a temperature used in the hyperthermia therapy.

The phase transition temperature of the liposome membrane prepared is reportedly near the weight average phase transition temperature of the saturated phospholipids used, but in the case of the present liposomes having a smaller average particle diameter, the phase transition temperature of the liposome membrane formed is lower than the weight average phase transition temperature of phospholipids used by about 1-4°C. Such a difference in phase transition temperature is proof of the difference of the present liposomes from conventional liposomes. According to an embodiment of the present invention, the molar ratio of DPPC and DSPC (DPPC/PSPC) is 90/10 to 50/50, preferably 85/15 to 65/35.

The present liposome formulation can contain a membrane stabilizer and a charge controlling agent so far as they do not adversely and severely affect the temperature sensitivity of the liposomes. Similarly, means for inhibiting the uptake of the liposomes by reticuloentherial tissues of the liver, spleen, and the like (for example, coating with water soluble polymers such as polyethyleneglycol or with gangliosides such as GM1) may be used.

Next, the drug-containing solutions to be entrapped in the liposomes in the present invention will be described. The osmotic pressure of the drug-containing solution is adjusted to an osmotic pressure 2.0 to 5.0 times, preferably 2.5 to 4.5 times, higher than that of body fluid of the warm-blooded animals. The above-defined osmotic pressure may be accomplished by a drug alone if the solubility of the drug is enough to obtain the desired osmotic pressure. Alternatively, if necessary, an appropriate osmotic pressure-controlling agent may be used. The osmotic pressure-controlling agents include but are not limited to, salts such as sodium chloride, sugars such as sucrose, amino acids such as glycine, and the like, which are water soluble and physiologically acceptable to warm-blooded animals.

Drugs used in the present invention are those wherein a synergetic effect in combination with the hyperthermia therapy is expected and a targeting effect is achieved by entrapping them in liposomes. Examples of the drugs are anticancer agents. Particular examples of the anticancer agents include metal complexes such as cisplatin (CDDP), carboplatin, tetraplatin, iproplatin etc.; anticancer antibiotics such as adriamycin, ansamitocin or a derivative thereof, bleomycin, Ara-C, daunomycin etc.; metabolic antagonists such as 5-FU, methotrexate; alkylating agents such as BCNU, CCNU etc.; other types of anticancer agents such as melphalan, mitoxantrone; and the like. Among them, a drug for which especially high targeting effect is expected is cisplation. See, Hyojunteki Chiryo Targetting Therapy) First edition, ed. T. Agishi, Japan Medical Center, Tokyo, Japan, 165 (1989); Y. Ogawa and H. Toguchi, Gan to Kagakuryoho (Cancer and Chemotherapy), 17, 1127 (1990).

Since the liposomes used in the present invention are SUVs, the volume needed for containing a drug is relatively small and the relative amount of lipids is somewhat large. However, since the toxicity of phospholipids composed with liposomes is generally low and the density of liposomes in a formulation can be controlled, a necessary dose can be achieved.

Next, the process for production of the present liposome formulation will be explained.

Any known processes for preparing SUVs, such as the ultrasonification method, French Press method, extrusion, and the like may be used. For example, in the ultrasonication method, first, a single phospholipid or a mixture of phospholipids having saturated acyl groups is dissolved in an organic solvent such as chloroform and thoroughly mixed. The solvent is then evaporated to form a thin layer of phospholipid. The thin layer is thoroughly dried, and a drug-containing solution is added. The whole is strongly agitated to form multilamellar vesicles, which are then treated with ultrasonification under a nitrogen flow to form SUVs. The liposome formulation thus obtained must be generally separated from the drug which has not been entrapped (free drug). For the separation of the free drug, gel filtration or dialysis is generally used, though in the case of separation of drug having an electric charge from SUVs not having the same electric charge, an ion-exchange resin is preferably used.

The present liposome formulation may be applied to any cancer carriers including human patients and animals, for which the hyperthermia therapy is applicable.

EXAMPLES

Next, the present invention will be further explained by the examples, comparative examples, and experiments.

Example 1

A solution of 21 $\mu$ moles DPPC and 9 $\mu$ moles DSPC in chloroform was placed into a 50 ml eggplant-shaped flask, and thoroughly mixed. The solvent chloroform was evaporated to form a thin layer of the phospholipids. The thin layer was thoroughly dried, and 2 ml of a solution of 0.094M calcein and 0.200M sucrose (pH 7.0) having an osmotic pressure of two times the physiological osmotic pressure was added thereon. The whole was strongly agitated to form multilamellar vesicles. The multilamellar vesicles were further treated by ultrasonication in a probe type ultrasonicator (Cell Disruptor 185, from BRANSON SONIFIER) under a nitrogen flow for 30 minutes to form SUVs. Next, the SUV preparation was passed through a diethylaminoethyl Sephadex A-25 column saturated with a sodium chloride-phosphate buffer (pH 7.4) having the same osmotic pressure as that of the solution entrapped in the liposomes, to adsorb and eliminate calcein not entrapped in the liposomes.

The average particle diameter of the SUVs thus prepared was less than 0.10 $\mu$m as determined by the light scattering method using a NICOMP model 370 from Particle Siging System Co. Ltd..

Example 2

The same procedure as described in Example 1 was repeated except that the solution entrapped in the liposomes comprised 0.094M calcein-0.400M sucrose (pH 7.4) and had an osmotic pressure 3.0 times higher than the physiological osmotic pressure. The average particle diameter of the liposomes thus prepared was less than 0.10 $\mu$m.

Example 3

The same procedure as described in Example 1 was repeated except that the solution entrapped in the liposomes comprised 0.094M calcein-0.560M sucrose (pH 7.4), and had an osmotic pressure 4.0 times higher than the physiological osmotic pressure. The average particle diameter of the liposomes thus prepared was less than 0.10 $\mu$m.

Example 4

The same procedure as described in Example 1 was repeated except that the solution entrapped in liposomes comprised 0.094M calcein-0.705M sucrose (pH 7.4) and had an osmotic pressure 5.0 times higher than the physiological osmotic pressure. The average particle diameter of the liposomes thus prepared was less than 0.10 $\mu$m.

Example 5

A solution of 42 $\mu$moles DPPC and 18 $\mu$moles DSPC in chloroform was placed into a 50 ml pear-shaped flask, and thoroughly mixed. The solvent chloroform was then evaporated to form a thin layer of the phospholipids. The thin layer was thoroughly dried, and 4 ml of a solution of 0.2% cisplatin (CDDP), 0.9% sodium chloride and 0.234M sucrose (pH 5.1) having an osmotic pressure 2 times higher than the physiological osmotic pressure was added thereon. The whole was strongly agitated to form multilamellar vesicles. The multilamellar vesicles were further treated by ultrasonication in a probe type ultra sonicator (Cell Disruptor 185, from Branson Sonifier), under a nitrogen flow for 30 minutes to form SUVs. Next, the SUV preparation was passed through a sulfopropyl Sephadex C-25 column saturated with a sodium chloride- phosphate buffer (pH 5.0) having the same osmotic pressure as that of the solution entrapped in the liposomes, to adsorb and eliminate the CDDP not entrapped in the liposomes.

The average particle diameter of the SUVs thus prepared was less than 0.10 μm as determined by the light scattering method using the NICOMP model 370.

Example 6

The same procedure as described in Example 5 was repeated to prepare a liposome formulation except that the solution entrapped in the liposomes comprised 0.2% CDDP-0.9% sodium chloride-0.439M sucrose (pH 5.0) and had an osmotic pressure 3.0 times higher than the physiological osmotic pressure. The average particle diameter of the liposomes thus prepared was less than 0.10 μm.

Example 7

The same procedure as described in Example 5 was repeated to form a liposome formulation except that the solution entrapped in the liposomes comprised 0.2% CDDP, 0.9% sodium chloride, and 0.620M sucrose solution (pH 5.0) and had an osmotic pressure 4.0 times higher than the physiological osmotic pressure. The average particle diameter of the liposomes thus prepared was less than 0.10 μm.

Example 8

The same procedure as described in Example 5 was repeated to prepare a liposome formulation except that the phospholipid solution in chloroform contained 48 μmoles DPPC and 12 μmoles DSPC. The solution entrapped in the liposomes had an osmotic pressure 2 times higher than the physiological osmotic pressure. The average particle diameter of the liposomes was less than 0.10 μm.

Example 9

The same procedure as described in Example 5 was repeated to prepare a liposome formulation except that the phospholipid solution in chloroform contained 48 μmoles DPPC and 12 μmoles DSPC, and the solution entrapped in the liposome comprised 0.2% CDDC-0.9% sodium chloride-0.439M sucrose (pH 5.0) and had an osmotic pressure 3.0 times higher than the physiological osmotic pressure. The average particle diameter of the liposomes thus prepared was less than 0.1 μm.

Example 10

The same procedure as described in Example 5 was repeated to prepare a liposome formulation except that the phospholipid solution in chloroform contained 48 μmoles DPPC and 12 μmoles DSPC, and the solution entrapped in the liposomes comprised 0.2% CDDP-0.9% sodium chloride-0.620M sucrose solution (pH 5.0) and had an osmotic pressure 4.0 times higher than the physiological osmotic pressure. The average particle diameter of the liposomes thus prepared was less than 0.1 μm.

Comparative Example 1

The same procedure as described in Example 1 was repeated to prepare a liposome formulation except that the solution entrapped in the liposomes comprised 0.094M Calcein (pH 7.4) and had an osmotic pressure 1.1 times higher than the physiological osmotic pressure. The average particle diameter of the liposomes thus prepare0d was less than 0.10 μm.

Comparative Example 2

The same procedure as described in Example 5 was repeated to form a liposome formulation except that the solution entrapped in the liposome comprised 0.2% CDDP and 0.9% sodium chloride (pH 5.0) and had an osmotic pressure the same as the physiological osmotic pressure. The average particle diameter of the liposomes thus prepared was less than 0.10 μm.

Experiment 1

A temperature sensitivity test was carried out for the liposome formulations prepared in Examples 1 to 4 and Comparative Example 1, using Calcein as an indicator.

Each liposome formulation was diluted with sodium chloride buffer (pH 7.4) having the same osmofic pressure as that of the entrapped solution in SUV so that the concentration in the diluted solution of the drug entrapped in the liposomes per the total volume of the diluted solution became to 167 μM. The diluted solution was used for the temperature sensitivity test. Namely, 0.5 ml of blood obtained from female Crj:CD-1 mice was pre-incubated for 5 minutes at a temperature of 37°C, 41°C, or 43°C, and 50 μl of the diluted solution was added thereon. After 2, 5, 10, 15, 20, and 30 minutes from the addition of the diluted liposome solution, 10 μl each of samples were obtained and added to 1.2 ml of a buffer solution (0.35 M sucrose-0.01 M EDTA-0.04M Tris HCl, pH 8.5) for fluorometry. Immediately after that, the sample was centrifuged at 1400 g for 10 minutes to eliminate blood cells. After 0.9 ml of the supernatant was used to determine a fluorescence (Fluorescence A), 0.1 ml of 10% Triton x 100 solution was added to the supernatant to completely disrupt the liposome in order to release Calcein, and fluorescence (Fluorescence B) was measured. The ratio of the release of the drug was calculated according to the equation (1).

$$\text{Release ratio} = \{1 - (0.9 \times A/B)/\text{self-quenching value}\} \times 100 \qquad (1)$$

The self-quenching value of liposome is a value obtained for the buffer solution prior to the addition of the blood sample. The self-quenching value was obtained by dividing the fluorescence of a 0.9 ml buffer solution by the fluorescence measured after the addition of 0.1 ml of 10% Triton × 100 solution.

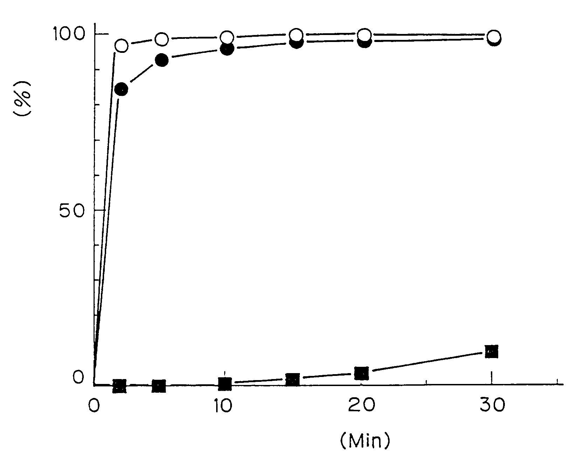

The releasing rate after 15 minute incubation at. different temperatures are summarized in Table 1. Moreover, Fig. 1 shows the change of the releasing ratios along with the elapse of time for the liposome formulation prepared in Example 3 as representative of the formulations of the present invention. In the Fig. 1, -■-, -●- and -○- show the results at 37°C, 41°C, and 43°C respectively.

Table

| Releasing Ratio of Calcein at Different Temperatures | | | | |
|---|---|---|---|---|
| | Osmotic pressure (times) | Releasing rate (%) | | |
| | | 37°C | 41°C | 43°C |
| Comparative Example 1 | 1.1 | 33.9 | 96.8 | 97.2 |
| Example 1 | 2.0 | 13.6 | 97.2 | 98.2 |
| Example 2 | 3.0 | 6.0 | 97.2 | 98.6 |
| Example 3 | 4.0 | 1.7 | 97.4 | 99.7 |
| Example 4 | 5.0 | 6.2 | 98.4 | 99.4 |

As seen from the Table 1, where the osmotic pressure of the solution entrapped in the liposomes is lower than 2.0 times the physiological osmotic pressure, the membrane of the liposomes at 37°C is very unstable and the entrapped drug is released, but the release of the drug is decreased by the rise of the osmotic pressure of the entrapped solution. In addition, the releasing ratios at 43°C are consistently excellent, showing that the present liposome preparation is useful for hyperthermia therapy. Moreover, the releasing ratios at 41°C are similarly high, and as shown in Fig. 1, the present liposomes are disrupted immediately after the addition thereof, showing that the present liposome formulation is highly temperature sensitive.

Experiment 2

A temperature sensitivity test was carried out for the liposome formulations prepared in Examples 5 to 10 and Comparative Example 2, using cisplatin as an indicator.

The concentration of CDDP entrapped in the liposome per total volume was adjusted to 400 μM with sodium chloride buffer (pH 5.0) having the same osmatic pressure as that of the entrapped solution is SUV. The diluted solution was used for the temperature sensitivity test. Namely, 0.582 ml of blood obtained from female Crj:CD-1 mice was incubated at 37°C for 5 minutes, and 100 μl of the diluted liposome solution was added thereon. After 2, 5, 10, 15, 20, and 30 minutes from the addition of the liposome solution, 60 μl each of samples were obtained and added to 0.15 ml of a sodium chloride- phosphate buffer solution (pH 5.0) having the same osmotic pressure as that of the solution entrapped in SUV. Immediately after that, the mixture was centrifuged at 1400 × g for 10 minutes to separate blood cells. 50 μl of the supernatant was put into a test tube (Sample T), and the remaining supernatant was transferred to a Centricon 100 (Grace Japan, Amicon Department) and centrifuged at 170 g for 20 minutes to obtain a filtrate not containing liposomes (Sample F). To the Samples F and T was added 0.2 ml of concentrated nitric acid. The mixtures were heated at 140°C for 2 minutes. In addition, 0.2 ml of about 30% hydrogen peroxide was added thereon and each mixture was heated at 140°C for 30 minutes for wet ashing to obtain a clear sample solution. After measuring the volume of the solutions, amounts of platinum in the samples F and T were quantitated using an atomic absorption spectrophotometer (Type Z-9000, Hitachi). The releasing rate of CDPP at 37°C ($R_{37}$) was calculated according to the following equation (2):

$$R_{37} = PtF/PtT \tag{2}$$

In the equation, PtF shows the amount of platinum in the Sample F, and PtT shows the amount of platinum in the Sample T.

Incubation tests at 41°C and 43°C were carried out according to the same procedure as described above except that samples were obtained after 1, 2, 4, 7, 10 and 15 minutes from the addition of the liposome solution. The releasing ratios of cisplatin at 41°C and 43°C ($R_{41}$ and $R_{43}$) were calculated according to the equation (3):

$$R_{41} \text{ or } R_{43} = PtF/PtT/0.77 \tag{3}$$

In the formula (3), the value 0.77 is a correcting factor obtained from a free cisplatin solution not entrapped in liposomes using the same incubation test as described above.

The releasing rate after 15 minute/incubation at different temperatures are summarized in Tables 2 and 3.

Table 2

| Release of Cisplatin from TS-SUV | | | | |
|---|---|---|---|---|
| Sample | Osm. press. | Release (%) | | |
| | | 37°C | 41°C | 43°C |
| Reference 2 | 1.0 | 17.0 | 56.5 | 56.6 |
| Example 5 | 2.0 | 17.3 | 67.1 | 88.5 |
| Example 6 | 3.0 | 12.5 | 63.5 | 91.8 |
| Example 7 | 4.0 | 6.7 | 50.9 | 86.9 |
| TS-SUV (temperature sensitive small unilamellar vesicle). | | | | |

Table 3

| Release of Cisplatin from TS-SUV | | | | |
|---|---|---|---|---|
| Sample | Osm. press. | Release (%) | | |
| | | 37°C | 41°C | 43°C |
| Reference 3 | 1.0 | 26.0 | 56.5 | 75.4 |
| Example 8 | 2.0 | 15.3 | 69.1 | 79.5 |
| Example 9 | 3.0 | 10.1 | 72.6 | 92.7 |
| Example 10 | 4.0 | 11.7 | 65.3 | 81.3 |
| TS-SUV (temperature sensitive small unilamellar vesicle). | | | | |

As seen from Tables 2 and 3, liposomes entrapping a drug solution having the same osmotic pressure as the physiological osmotic pressure release a significant amount of the drug at 37°C and do not provide excellent release of the drug at 41°C and 43°C, revealing less temperature sensitivity. On the other hand, for the present liposome formulations entrapping a hypertonic drug solution, the release rate of the CDDP at 37°C is decreased as the osmotic pressure increases, and a higher release rate of the CDDP is provided at 41°C and 43°C.

Experiment 3

Cancer cells S-180 were implanted in a left foot muscle of Crj:CD-1 mice 7 weeks old, and on 9th day after implantation 20 cancer bearing mice which cancer tissues grew to 0.53 cm of mean diameter (a diameter of the left foot beating cancer minus a diameter of the normal right foot) were selected and divided 5 groups. The cancer diameters were distributed as narrow as a coefficient of variation value was 4.0%. Hyperthermia therapy was carried out as follow. A coated thermocouple was attached to the akin surface of a cancer tissue to take a skin temperature signal, which was then transmitted to a temperature controller. The temperature controller regulated the output of an alternating regulated DC power supply which heated a cord heater wrapping the whole cancer tissue. In this manner, a target portion was constantly heated at 42.5 ± 0.1°C.

SUV containing CDDP, final concentration of which was adjusted to 400 µM with sodium chloride buffer (pH 5.0) was prepared in Example 8 manner and used as injection solution. Free 400 µM CDDP solution (not entrapped in a SUV) was prepared by dilution of a Randa Injection (Nippon Kayaku Corp.) with isotonic sodium chloride solution. 1.5 mg/kg of CDDP was injected from a tail vein. Immediately after the injection heating was started, and after akin temperature equaled 42.5°C, the heating was further continued for 40 minutes. The treatment was carried out three times on 9th, 13th and 17th days after implantation.

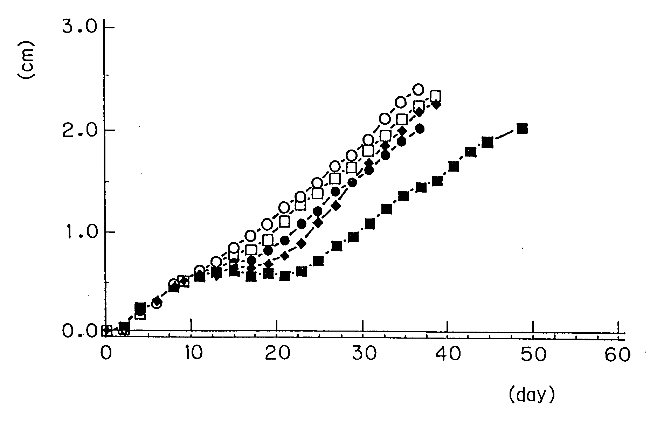

Figure 2 show the result. In Fig. 2, -■- shows a result of the group wherein CDDP- entrapping SUVs were intravenously injected and the hyperthermia therapy was applied; -□- shows a result of the group wherein CDDP-entrapping SUVs were intravenously injected and the hyperthermia therapy was not applied; -♦- shows a result of the group wherein CDDP aqueous solution was intravenously injected and the hyperthermia therapy was applied; -o- shows a result of the group wherein only the hyperthermia therapy was applied; and -•- shows a result of the group wherein no treatment was applied.

As seen from Fig. 2, the group wherein the CDDP-entrapping SUVs were injected and the hyperthermia therapy was applied (-■-) exhibited an anticancer effect. This effect was significantly higher than that of the group wherein CDDP aqueous solution was injected and the hyperthermia therapy was applied (-♦-). An average cancer diameter after 20 days when all mice from the day of the final treatment for the group -■- and the group -♦- was 58% and 91% respectively in comparison to the control group -o-.

## Claims

1. A liposome formulation comprising liposomes entrapping a drug-containing solution having an osmotic pressure 2.0 to 5.0 times higher than the physiological osmotic pressure, said liposome being small unilamellar vesicle liposomes having an average particle diameter of not more than 0.1 µm and having a phase transfer temperature of the membrane of 40°C to 45°C.

2. A liposome formulation according to claim 1, wherein the main component of the liposome membrane is a phospholipid having saturated acyl groups.

3. A liposome formulation according to claim 1, wherein the osmotic pressure of the drug-containing solution is 2.5 to 4.5 times higher than the physiological osmotic pressure.

4. A liposome formulation according to claim 1, wherein the drug is an anticancer agent.

5. A process for production of a liposome formulation comprising the steps of:

   (1) preparing a drug-containing solution having an osmotic pressure 2.0 to 5.0 times higher than the physiological osmotic pressure;
   (2) preparing a thin layer of a phospholipid;
   (3) mixing the drug-containing solution and the thin layer of phospholipid to form multilamellar vesicle liposomes; and
   (4) sonicating the multilamellar vesicle liposomes to form small unilamellar vesicle liposomes comprising the drug-containing solution having an osmotic pressure 2.0 to 5.0 times higher than the physiological osmotic pressure entrapped in the small unilamellar vesicle liposomes having an average particle diameter of not more than 0.1 µm and having a phase transfer temperature of the membrane of 40°C to 45°C.

6. A process according to claim 5, wherein the main component of the liposome membrane is a phospholipid having saturated acyl groups.

7. A process according to claim 5, wherein the drug is an anticancer agent.

**Patentansprüche**

1. Eine Liposomen aufweisende Liposomformulierung, welche eine arzneimittelhaltige Lösung einschließen, welche einen 2,0 bis 5,0-fach höheren osmotischen Druck aufweist als den physiologischen osmotischen Druck, wobei die Liposomen kleine unilamellare Vesikel-Liposomen sind, welche einen mittleren Partikeldurchmesser von nicht mehr als 0,1 μm besitzen und eine Phasenübergangstemperatur der Membran von 40°C bis 45°C besitzen.

2. Eine Liposomformulierung gemäß Anspruch 1, bei welcher die Hauptkomponente der Liposommembran ein Phospholipid mit gesättigten Acylgruppen ist.

3. Eine Liposomformulierung gemäß Anspruch 1, bei welcher der osmotische Druck der arzneimittelhaltigen Lösung 2,5 bis 4,5-fach höher ist als der physiologische osmotische Druck.

4. Eine Liposomformulierung gemäß Anspruch 1, bei welcher das Arzneimittel ein Antikrebsmittel ist.

5. Ein Verfahren zur Herstellung einer Liposomformulierung, welche die Schritte aufweist:

   (1) Ansetzen einer arzneimittelhaltigen Lösung mit einem 2,0 bis 5,0-fach höheren osmotischen Druck als den physiologischen osmotischen Druck;

   (2) Präparation einer dünnen Schicht eines Phospholipids;

   (3) Vermischen der arzneimittelhaltigen Lösung und der dünnen Phospholipidschicht, um multilamellare Vesikel-Liposomen auszubilden; und

   (4) Beschallen der multilamellaren Vesikel-Liposomen, um kleine unilamellare Vesikel-Liposomen auszubilden, welche eine arzneimittelhaltige Lösung mit einem 2,0 bis 5,0-fach höheren osmotischen Druck als den physiologischen osmotischen Druck aufweisen, welche in den kleinen unilamellaren Vesikel-Liposomen eingeschlossen ist, die einen mittleren Partikeldurchmesser von nicht mehr als 0,1 μm besitzen und eine Phasenübergangstemperatur der Membran von 40°C bis 45°C besitzen.

6. Ein Verfahren gemäß Anspruch 5, bei dem die Hauptkomponente der Liposommembran ein Phospholipid mit gesättigten Acylgruppen ist.

7. Ein Verfahren gemäß Anspruch 5, bei dem das Arzneimittel ein Antikrebsmittel ist.

**Revendications**

1. Formulation liposomique, comprenant des liposomes emprisonnant une solution contenant un médicament ayant une pression osmotique 2,0 à 5,0 fois supérieure à celle de la pression osmotique physiologique, les liposomes en question étant des liposomes vésiculaires unilamellaires de petite dimension ayant un diamètre moyen de particule non supérieur à 0,1 μm et ayant une température de transfert de phase de la membrane de 40 à 45°C.

2. Formulation liposomique suivant la revendication 1, dans laquelle le composant principal de la membrane liposomique est un phospholipide portant des groupes acyle saturés.

3. Formulation liposomique suivant la revendication 1, dans laquelle la pression osmotique de la solution contenant le médicament est 2,5 à 4,5 fois supérieure à la pression osmotique physiologique.

4. Formulation liposomique suivant la revendication 1, dans laquelle le médicament est un agent anti-cancéreux.

5. Procédé de production d'une formulation liposomique, qui comprend les étapes consistant :

   (1) à préparer une solution contenant un médicament ayant une pression osmotique 2,0 à 5,0 fois supérieure à la pression osmotique physiologique ;
   (2) à préparer une mince couche d'un phospholipide ;
   (3) à mélanger la solution contenant le médicament et la mince couche de phospholipide pour former des liposomes vésiculaires multilamellaires ; et

(4) à soumettre à l'action des ultrasons les liposomes vésiculaires multilamellaires pour former des liposomes vésiculaires unilamellaires de petite dimension comprenant la solution contenant le médicament ayant une pression osmotique 2,0 à 5,0 fois supérieure à la pression osmotique physiologique emprisonnée dans les liposomes vésiculaires unilamellaires de petites dimensions ayant un diamètre moyen de particule ne dépassant pas 0,1 μm et ayant une température de transfert de phase de la membrane de 40 à 45°C.

6. Procédé suivant la revendication 5, dans lequel le composant principal de la membrane liposomique est un phospholipide portant des groupes acyle saturés.

7. Procédé suivant la revendication 5, dans lequel le médicament est un agent anticancéreux.